# EUROPEAN PATENT SPECIFICATION

(11) **EP 0 835 139 B1**
(45) Date of publication and mention of the grant of the patent: **03.09.2003**
(21) Application number: 96920653.1
(22) Date of filing: 28.06.1996
(51) Int. Cl.: A61K 49/00, A23L 1/09, A23L 1/308, A23L 1/164

(54) **SOLID ORAL DIAGNOSTIC TEST MEAL AND METHODS OF USE THEREOF**
ORALE FESTE DIAGNOSTISCHE MAHLZEIT UND VERFAHREN FÜR IHRE VERWENDUNG
REPAS D'EPREUVE SOLIDE DE DIAGNOSTIC ORAL, ET PROCEDES D'UTILISATION

(30) Priority: 30.06.1995 US 497508; 26.06.1996 US 670709
(43) Date of publication of application: 15.04.1998
(73) Proprietor: Ceapro Inc., Edmonton, Alberta T5J 3S4 (CA)
(72) Inventor: PALMASON, Carol, Calgary, Alberta T2S 1G3 (CA); WOLEVER, Thomas M.S., Toronto, Ontario M6P 2L8 (CA)
(74) Representative: Fritzsche, Thomas M., Dr.
(86) International application number: CA9600434
(87) International publication number: WO97002050

(56) References cited:
- US-A- 5 472 952
- NUTR. RES. (N. Y.) (1996), 16(6), 899-905 CODEN: NTRSDC;ISSN: 0271-5317, 1996, XP002025712 WOLEVER, THOMAS M. S. ET AL: "Less variation of postprandial blood glucose after starchy test meals than oral glucose"
- DEUTSCHE MEDIZINISCHE WOCHENSCHRIFT, vol. 112, no. 51, 1987, pages 1977-1983, XP002025713 W. SICHERT-OEVERMANN : "BLUTGLUCOSE- UND INSULINVERLAUF BEI GESUNDEN UND DIABETIKERN NACH GABE ROHER VOLLKORNZUBEREITUNGEN, INSBESONDERE FRISCHKORNMÜSLI."
- CARBOHYDRATE POLYMERS, vol. 21, 1993, BARKING GB, pages 189-194, XP002025714 C. DUBOIS: "CEREAL DIETARY FIBERS AFFECT POST-PRANDIAL LIPOPROTEINS IN HEALTHY HUMAN SUBJECTS"
- CHEMICAL ABSTRACTS, vol. 125, no. 15, 7 October 1996 Columbus, Ohio, US; abstract no. 194285, FAIRCHILD, R. M. ET AL: "A new breakfast cereal containing guar gum reduces postprandial plasma glucose and insulin concentrations in normal-weight human subjects" XP002025718 & BR. J. NUTR. (1996), 76(1), 63-73 CODEN: BJNUAV;ISSN: 0007-1145, 1996,
- CHEMICAL ABSTRACTS, vol. 125, no. 13, 23 September 1996 Columbus, Ohio, US; abstract no. 166493, GUEVIN, NATHALIE ET AL: "Postprandial glucose, insulin, and lipid responses to four meals containing unpurified dietary fiber in non- insulin -dependent diabetes mellitus (NIDDM), hypertriglyceridemic subjects" XP002025719 & J. AM. COLL. NUTR. (1996), 15(4), 389-396 CODEN: JONUDL;ISSN: 0731-5724, 1996,
- CHEMICAL ABSTRACTS, vol. 98, no. 3, 17 January 1983 Columbus, Ohio, US; abstract no. 11290, FOELSCH, U. R. ET AL: "Effect of acarbose and fiber on glucose and hormone response to sucrose load or mixed meal" XP002025720 & INT. CONGR. SER. - EXCERPTA MED. (1982), 594, 97-106 CODEN: EXMDA4;ISSN: 0531-5131, 1982,
- BR. J. NUTRITION, vol. 71, 1994, pages 675-685, XP002025715 C. CHERBUT: "INVOLVEMENT OF SMALL INTETSINAL MOTILITY IN BLOOD GLUCOSE RESPONSE TO DIETARY FIBRE IN MAN."
- EUR. J. CLIN. NUTRITION, vol. 42, 1988, pages 519-526, XP002025716 B. KARLSTRÖM: "EFFECTS OF FOUR MEALS WITH DIFFERENT KINDS OF DIETARY FIBRE ON GLUCOSE METABOLISM IN HEALTHY SUBJECTS AND NON-INSULIN-DEPENDENT DIABETIC PATIENTS."

## Description

### Technical Field

The present invention relates generally to a use of products and kits in metabolic diagnostic tests. In particular, the invention relates to a use of a solid oral diagnostic test meal and a use of a kit for determining the postprandial concentration of a blood constituent in a vertebrate subject.

### Background

Disorders of carbohydrate metabolism traditionally include diabetes mellitus and impaired glucose tolerance. More recently, insulin resistance has been recognized as a disorder of carbohydrate metabolism that leads to raised plasma insulin concentrations. Raised plasma insulin has been suggested to contribute to the pathogenesis of diabetes, hypertension, raised serum triglycerides, reduced serum HDL cholesterol and atherosclerosis.

Diabetes is a well-recognized cause of significant morbidity and mortality. With a prevalence in North America which will approach 10% in the next decade, non-insulin dependent diabetes ("NIDDM") is a major public health concern.

NIDDM is the most common diagnosis of patients entering dialysis programs in the United States, a major cause of vision loss and a major contributing factor in cardiac, peripheral and cerebral vascular diseases. Although there is no direct evidence in NIDDM, the success of the Diabetes Control and Complications trial in showing that improving glucose control markedly reduced the development of diabetes complications in insulin-dependent diabetic patients, suggests that early diagnosis and treatment of NIDDM to reduce hyperglycemia may reduce complications.

Glucose tolerance in a patient is determined by orally administering a standard amount of glucose and monitoring the responsive blood glucose levels over time. For example, when a normal fasting person ingests 50 grams of glucose, his blood glucose level rises from a fasting level of approximately 90 mg/dL to approximately 140 mg/dL and falls back to the fasting level within about three hours. A diabetic person typically has a fasting blood glucose level above 120 mg/dL. On ingesting 50 grams of glucose, a diabetic exhibits a rise in blood glucose levels for about two to three hours and the blood glucose level falls back to the diabetic fasting level after about five to six hours or more. It has been reported that the postprandial blood glucose response in subjects with normal or abnormal carbohydrate metabolism can be blunted by consumption of a high-carbohydrate, high-fiber bar (McIvor et al. (1985) *Diabetes Care* 8:274-278).

A patient with impaired glucose tolerance ("IGT") displays an abnormal glucose tolerance in which the blood glucose levels are not high enough to be associated with the specific complications of diabetes. However, despite the fact that IGT is a major risk factor for the development of NIDDM, peripheral vascular disease and cardiovascular disease, widespread screening for IGT and asymptomatic NIDDM has not been routinely conducted. Furthermore, screening for hyperinsulinemia and postprandial blood insulin levels is currently only done for research purposes. Screening may become more common if easier and less expensive methods become available.

The internationally accepted method of diagnosing diabetes, IGT, NIDDM and hyperinsulinemia is to administer a 75-gram oral glucose tolerance test ("OGTT"). The main reason for the use of this test is that a postprandial blood glucose value is needed to make an accurate diagnosis; the use of fasting glucose alone lacks adequate specificity and sensitivity (Modan et al. (1994) *Diabetes Care* 17:436-439). Glucose is used because it is easy to standardize the amount administered, it is easily stored and its absorption is not influenced by other food factors such as protein or fat, cooking and processing. However, the OGTT has drawbacks and is not commonly done as a clinical test. Thus, diabetes, IGT and hyperinsulinemia are not generally diagnosed early.

One of the drawbacks of the OGTT is the difficulty of the test, which requires a 12- to 16-hour fast and a timed blood sample. In addition, glucose is generally unpalatable and 75 grams is a large dose that may lead to nausea and other gastrointestinal side-effects. Moreover, the results of the test are highly variable often leading to false positives and false negatives.

The diagnosis of diabetes is established if fasting venous plasma glucose at least 140 mg/dL (7.8 mmol/L) or plasma glucose is at least 200 mg/dL (11.1 mmol/L) two hours after the ingestion of an oral 75-gram glucose load (National Diabetes Group (1979) *Diabetes* 28:1039-1057); WHO Expert Committee (1980) Diabetes *Mellitus*, Geneva, World Health Organization (Tech. Rep. Ser. no. 646)), as is customarily performed in an OGTT. These values differ slightly if venous whole blood or capillary whole blood samples are used. In 334 subjects who took repeated 100-gram OGTTs, the median 2-hour postprandial plasma glucose concentration and within-subject standard deviation ("SD") thereof were 84.0 mg/dL and 12.2 mg/dL, respectively (McDonald et al. (1965) *Diabetes* 14:473-480). The coefficient of variation (SD/mean x 100) of these data was about 14.5%. These data indicate the high variability of the results obtained from an OGTT. Because of this variability it is difficult to interpret the results of an OGTT.

In addition, glucose is a non-physiological test meal and stimulates a relatively weak postprandial insulin response compared to the oral glucose-stimulated postprandial blood glucose response. Although the blood glucose response of starchy foods is less than that of glucose, the insulin response to starchy foods is greater, possibly because of the presence of fat and protein (Bornet et al. (1987) *Am. J. Clin. Nutr.* 45:588-595; Chew et al. (1988) 47:53-56; Indar-Brown et al (1992) *Am. J. Clin*. *Nutr*. 55:89-95). Accordingly, postprandial insulin responses are not routinely measured and cannot be accurately screened using an oral administration of liquid glucose.

It is clear that near-normal glycemic control can prevent diabetic complications. However, early interest in mass screening for diabetes to facilitate early diagnosis and prevent debilitating or fatal complications was tempered by the undesirable economic, social, physical and psychological consequences of diagnosing diabetes. The current absence of a successfully implemented diabetes mass screening program is the result of the lack of resolution of these concerns (Harris et al. (1994) *Diabetes Care.* 17:440-445; Knowler (1994) *Diabetes Care* 17:445-450).

Current interest in diabetes screening programs have fostered studies on, for example, random capillary blood glucose measurements (Engelgau et al. (1995) *Diabetes Care* 18:463-466) and questionnaires to evaluate the prevalence of diabetes risk factors and thereby prospectively identify subjects at increased risk for undiagnosed diabetes (Herman et al. (1995) *Diabetes Care* 18:382-387). These tests have proven to be variable, empirical and qualitative. Selecting those subjects from the general population who present risk factors merely identifies candidates for more precise and quantitative tests.

B. Karlström et al. describes in EUR. J. CLIN. NUTRITION, vol. 42, 1988, pages 519-526 the effects of four meals with different kinds of dietary fibre on glucose metabolism in healthy subjects and non-insulin-dependent diabetic patients.

Accordingly, there is a need in the art for a standardized, physiologically balanced diagnostic test meal that can be used as a diagnostic tool as well as a tool to assist in the management of disorders of carbohydrate metabolism.

### Summary

Central to the present invention is the discovery that the novel solid diagnostic test meal disclosed herein produces a more precise measurement of postprandial glucose and insulin responses compared to liquid glucose beverages and liquid meals.

Accordingly, the present invention provides a use of a solid oral diagnostic test meal for determining the postprandial concentration of a blood constituent in a vertebrate subject. The test meal comprises
a) a complex carbohydrate that provides a quantity of available carbohydrate effective to increase blood glucose levels in a vertebrate subject when ingested by the subject, wherein the carbohydrate is present in an amount in the range of about 50% to about 65% of the energy provided in the meal;
b) a source of dietary fat, wherein the dietary fat is present in an amount in the range of about 15% to about 30% of the energy provided in the meal;
c) a source of dietary protein, wherein the dietary protein is present in an amount in the range of about 10% to about 25% of the energy provided in the meal; and optionally
d) a source of dietary fiber and flavoring.

Further, the invention is directed to the use of the test meal, wherein the test meal is used for diagnosing a disorder of carbohydrate metabolism, wherein the test meal is used for managing the dosage of a drug that decreases postprandial glucose concentration and wherein the test meal is used for self-monitoring and/or self-diagnosis of diabetes.

Further, the invention provides a use of a kit for determining the postprandial concentration of a blood constituent in a vertebrete subject comprising a solid oral diagnostic test meal comprising:
a) a complex carbohydrate that provides a quantity of available carbohydrate effective to increase blood glucose levels in a vertebrete subject when ingested by the subject, wherein the carbohydrate is present in an amount in the range of about 50% to about 65% of the energy provided in the meal;
b) a source of dietary fat, wherein the dietary fat is present in an amount in the range of about 15% to about 30% of the energy provided in the meal;
c) a source of dietary protein, wherein the dietary protein is present in an amount in the range of about 10% to about 25% of the energy provided in the meal; and, optionally
d) a source of dietary fiber and flavouring.

Further, the invention is directed to the use of the kit, wherein the kit is used for diagnosing a disorder of carbohydrate metabolism; wherein the kit is used for managing the dosage of a drug that decreases postprandial glucose concentration and wherein the kit is used for self-monitoring and/or self-diagnosis of diabetes.

### Brief Description of the Figures

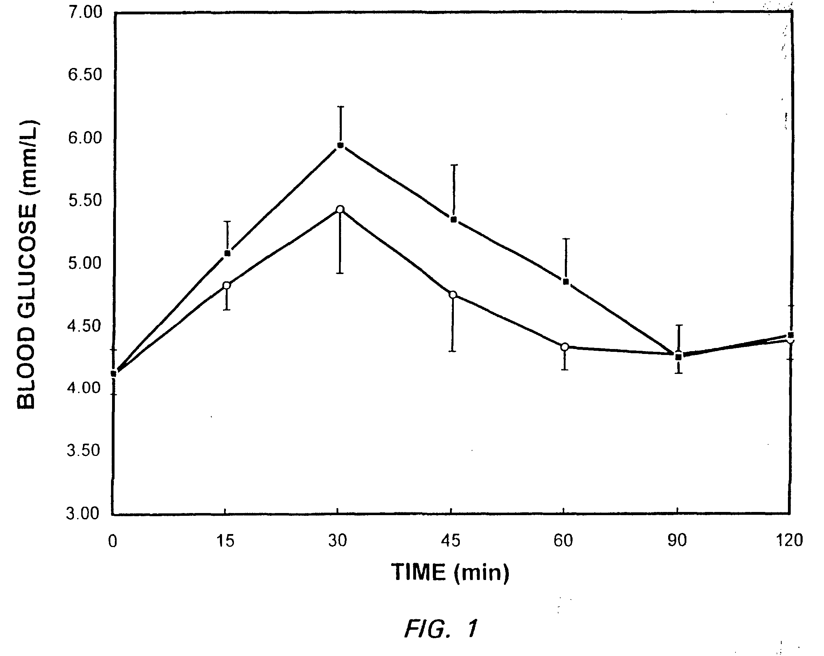

FIG. 1 is a graphical illustration of the postprandial blood glucose response in subjects after administration of acarbose (open symbols) or a placebo (closed symbols) and consumption of Ensure® as described in Example 6. The data points represent the mean blood glucose level in mmoles/L (mm/L), plus or minus the standard error of the mean, for six determinations at each time point.

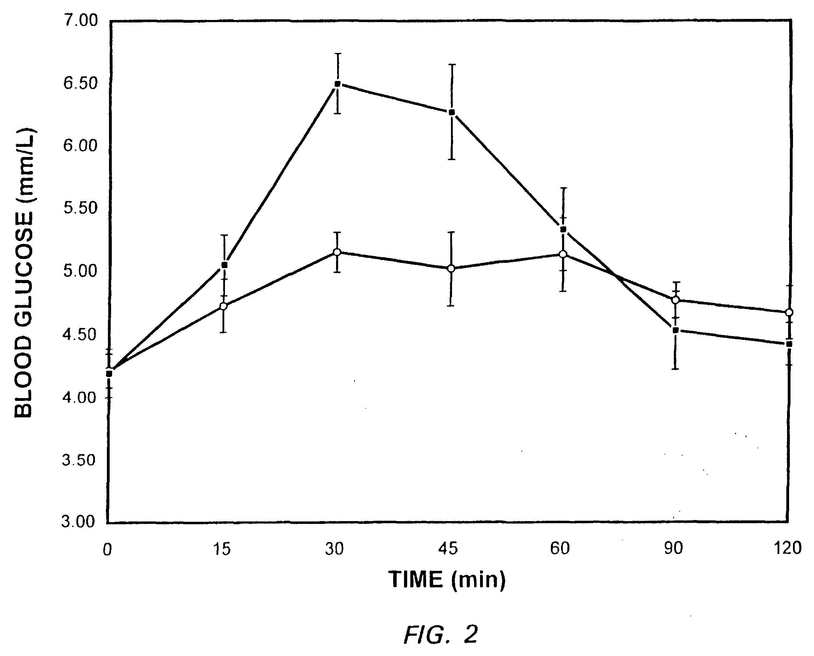

FIG. 2 is a graphical illustration of the postprandial blood glucose response in subjects after administration of acarbose (open symbols) or a placebo (closed symbols) consumption of a solid test meal as described in Example 6. The data points represent the mean blood glucose level in mmoles/L (mm/L), plus or minus the standard error of the mean, for six determinations at each time point.

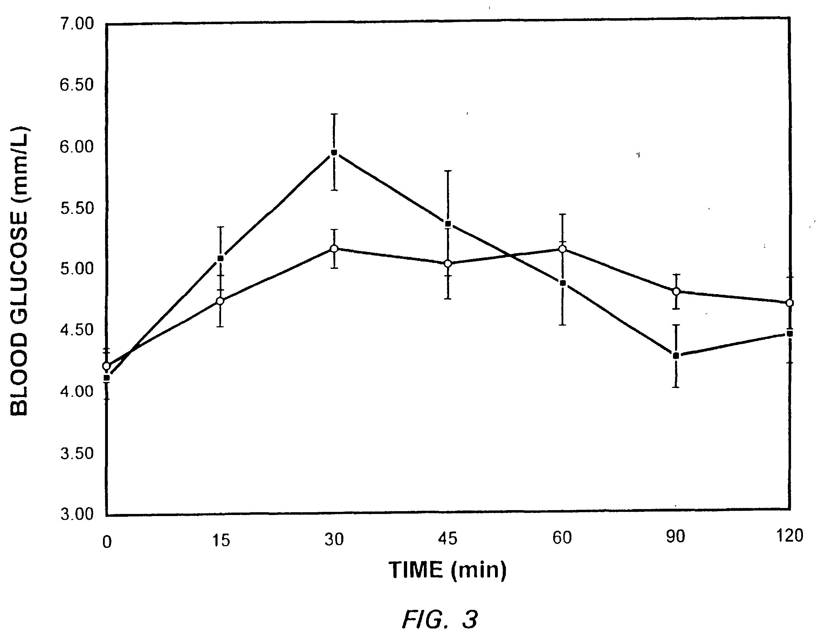

FIG. 3 is a graphical illustration of the postprandial blood glucose response in subjects after administration of acarbose and consumption of a solid test meal (open symbols) or Ensures (closed symbols) as described in Example 6. The data points represent the mean blood glucose level in mmoles/L (mm/L), plus or minus the standard error of the mean, for six determinations at each time point.

### Detailed Description

The practice of the present invention will employ, unless otherwise indicated, conventional methods of chemistry, biochemistry, medicine, nutritional analysis and food formulation, within the skill of the art. Such techniques are explained fully in the literature. *See, e.g*., Bergmeyer et al., eds. *Methods of Enzymatic Analysis*, Academic Press (New York), U.S. Dept. HEW (1982) *Lipid and Lipoprotein Analysis: Manual of Laboratory Operations, Lipid Research Clinics Program*, (Washington, DC), AOAC (1980) *Official Methods of Analysis* (Washington, DC), National Diabetes Group (1979) Classification and Diagnosis of Diabetes Mellitus and Other Categories of Glucose Intolerance, *Diabetes* 28:1039-1057, Furia (1972 and 1980) *Handbook of Food Additives*, 2d ed., Volumes I and II, CRC Press Inc. (West Palm Beach, FL), and Committee on Specifications, Committee on Food Protection, National Research Council (1980) *Food Chemicals Codex*, 3d ed., National Academy of Sciences (Washington, DC).

As used in this specification and the appended claims, the singular forms "a," "an" and "the" include plural references unless the content clearly dictates otherwise. Thus, the term "a blood glucose measurement" can include more than one such measurement.

### A. Definitions

In describing the present invention, the following terms will be employed, and are intended to be defined as indicated below. Although reference is made to blood as a biological sample in the following definitions, the term is intended to encompass other biological samples as well.

By "glycemic response" is meant the amount of glucose produced in the blood following ingestion of a food, test meal or the like taken at a specific postprandial time point, for example, 30, 60, 90 and/or 120 minutes. A subject's glycemic response may be expressed as a percentage the response to a control standard, for example, a 75-gram glucose-containing beverage, e.g., Glucodex® (Bougier, Inc., Camble, Quebec). Preferably the glycemic index is taken 60 minutes postprandial.

The "glycemic index" of a food, test meal or the like ("GI") is defined as (F/S) x 100 where F is the area under the curve ("AUC") of the glycemic response taken at multiple time points from 0 to 120 minutes after ingestion an amount of the food or test meal that provides 50 grams of available carbohydrate and S is the AUC of the glycemic response after ingestion of a reference food that provides 50 grams of available carbohydrate. Preferably the reference food is white bread or a liquid glucose beverage. In determining the glycemic index of a food or test meal, F and S are taken in a subject; preferably S is determined in triplicate and the mean value is used to calculate the GI. The GI of a food or test meal is preferably calculated as the mean of the GIs for that food or test meal that has been determined in about three to ten or more subjects.

The "area under the curve" ("AUC") used to calculate the glycemic index of a food or test meal is the incremental area under the curve of the glycemic response above fasting glucose levels. For example, the AUC can be calculated geometrically as follows, wherein Iₙ is the difference between the blood glucose level at time n and the fasting blood glucose level and Δt is the time interval between Iₙ and Iₙ₋₁. Blood samples are taken after a 10- to 16-hour fast and at n time points from 0 to 120 minutes or more after ingestion of the food or test meal has begun. AUC is the sum of Aₙ over *n* time points wherein: Aₙ = (Δt x (Iₙ + Iₙ₋₁))/2, when both Iₙ and Iₙ₋₁ are greater than 0; Aₙ = (Δt x (Iₙ)²)/(2 x (Iₙ - Iₙ₋₁), when Iₙ is greater than 0 and Iₙ₋₁ is less than 0; Aₙ = (Δt x (Iₙ₋ ₁)²)/(2 x (Iₙ₋₁ - Iₙ), when Iₙ is less than 0 and Iₙ₋₁ is greater than 0; and Aₙ = 0, when In and Iₙ₋₁ are less than 0.

The glycemic index expressed as AUC may be used as a basis for dietary carbohydrate exchange and as a reference for assessing the glucose response to a particular food. (*see,* Jenkins et al. (1981) *Am. J*. *Clin. Nutr.* 34:362-366, and Jenkins et al. (1983) *Diabetologia* 24:257-264). The glycemic index may be determined as described in Wolever et al. (1985) *Diabetes Care* 8:418-428, Wolever et al. (1991) *Am. J. Clin. Nutr.* 54:846-854 and Wolever et al. (1994) *Am*. *J. Clin. Nutr.* 59:1265-1269.

By "available carbohydrate" is meant the amount of total carbohydrate ingested which can be digested and absorbed. Total carbohydrate is calculated by difference, i.e., total carbohydrate ("TC") is calculated using the equation TC = S - M - A - F - P, where S is the weight of the food sample, M is the moisture content, A is the ash content, F is the fat content and P is the protein content. The methods for measuring moisture, ash, fat and protein are described in, e.g., *AOAC Official Methods of Analysis* (1980) Washington, DC, Association of Official Analytical Chemists. Available carbohydrate is the difference between total carbohydrate and dietary fiber in a food sample. Dietary fiber can be measured, e.g., according to the method of Prosky et al. (1988) *J. Assoc. Off. Anal. Chem.* 71:1017.

By "management of disorders of carbohydrate metabolism" is intended both the pharmacological diminution of postprandial blood glucose levels and/or insulin responses, a decrease in insulin resistance and a decrease in the medical sequelae which are the hallmark of such diseases, as well as the titration of drug dosage for the treatment of these diseases in individual subjects.

By "medically controlled" is meant that a diagnostic test meal contains a source of carbohydrate that provides a calibrated amount of available carbohydrate which, when administered to a subject, yields a predetermined glycemic response, preferably a predetermined glycemic index. Furthermore, "medically controlled" is intended to mean that the amount of available carbohydrate in a diagnostic test meal is periodically recalibrated to insure the precision of the predetermined response in test subjects and that the glycemic index is monitored to insure precision.

The term "insulin" includes both non-specific insulin and specific insulin. Insulin exists in various forms in the blood, which forms include insulin, proinsulin and a number of split proinsulin products. Non-specific insulin is determined using non-specific antibodies that cross-react with various forms of proinsulin. Specific insulin is determined using antibodies displaying little or no cross-reactivity with proinsulin. Such antibodies can be raised using routine methods well known in the art. Polyclonal serum or monoclonal antibodies can be prepared in response to the desired antigen.

Insulin, proinsulin or split-proinsulin or their fragments can be used to produce antibodies, both polyclonal and monoclonal. If polyclonal antibodies are desired, a selected mammal, (e.g., mouse, rabbit, goat, horse, etc.) is immunized with an antigen selected from insulin, proinsulin, split-proinsulin or fragments thereof. Serum from the immunized animal is collected and treated according to known procedures. If serum containing polyclonal antibodies is used, the polyclonal antibodies can be purified by immunoaffinity chromatography, using known procedures.

Monoclonal antibodies to insulin, proinsulin or split-proinsulin, and to fragments thereof, can be readily produced by one skilled in the art. The general methodology for making monoclonal antibodies by using hybridoma technology is well known. Immortal antibody-producing cell lines can be created by cell fusion, and also by other techniques such as direct transformation of B lymphocytes with oncogenic DNA, or transfection with Epstein-Barr virus. *See, e.g*., Schreier et al. (1980) *Hybridoma Techniques*; Hammerling et al. (1981) *Monoclonal Antibodies and T-cell Hybridomas*; Kennett et al. (1980) *Monoclonal Antibodies; see also* U.S. Patent Nos. 4,341,761; 4,399,121; 4,427,783; 4,444,887; 4,452,570; 4,466,917; 4,472,500, 4,491,632; and 4,493,890. Panels of monoclonal antibodies produced against the selected antigen can be screened for various properties; i.e., for isotype, epitope, affinity, etc.

A "reference concentration" of glucose or insulin in a biological specimen is that concentration that has been reported as normal for a normal, nonobese, nondiabetic, healthy subject. The reference concentration may be in reference to fasting glucose or insulin or in reference to postprandial glucose or insulin concentration. Such reference concentrations are described in, for example, National Diabetes Group (1979) *Diabetes* 28:1039-1057, and WHO Expert Committee (1980) *Diabetes Mellitus*, Geneva, World Health Organization (Tech. Rep. Ser. no. 646). In addition, a reference concentration may be an intra-subject reference, i.e., a concentration of glucose or insulin in a biological sample previously obtained from the subject being retested.

By "vertebrate subject" is meant any member of the subphylum chordata, including, without limitation, mammals such as cattle, sheep, pigs, goats, horses, and man; domestic animals such as dogs and cats; and birds, including domestic, wild and game birds such as cocks and hens including chickens, turkeys and other gallinaceous birds. The term does not denote a particular age. Thus, both adult and newborn animals are intended to be covered.

The terms "self-diagnosis", "self-teaching" and "self-monitoring" are meant to encompass the use of the diagnostic test meal in a non-clinic setting. Thus, for example, self-diagnosis is intended to encompass not only the use of the diagnostic test meal by a human subject on himself or herself for the diagnosis of a disorder of carbohydrate metabolism, but also the use of the diagnostic test meal by a party for the diagnosis of a disorder of carbohydrate metabolism in another vertebrate subject.

A "biological sample" may be derived from a variety of sources, e.g., human or other mammalian biological fluids or tissues, including blood (serum or plasma), urine, cerebrospinal fluid, stool, sputum, or wound or glandular exudates, ocular lens fluid, lymph fluid, genital washings, biopsy tissue samples, and the like. One preferred biological sample is blood.

The terms "blood glucose" and "blood insulin" are intended to mean glucose and insulin, respectively, measured in whole blood, serum, or plasma taken from a vein or a capillary bed. Typical sources of capillary blood include finger-, heel, or earlobe-pricks. One of ordinary skill in the art will recognize that the blood glucose level or blood insulin level will vary slightly depending on the source.

The glucose and insulin levels in a biological sample may be measured by any method known in the art. In this regard, glucose levels may be measured using the hexokinase method (Bergmeyer et al. (1974) in Bergmeyer et al., eds. *Methods of Enzymatic Analysis*, Academic Press (New York). Briefly, this assay entails simultaneously incubating the biological sample with the enzyme hexokinase, which catalyzes the transfer of the γ-phosphate group from adenosine triphosphate ("ATP") to glucose to form glucose-6-phosphate and glucose-6-phosphate dehydrogenase, which, in the presence of nicotinamide-adenine dinucleotide phosphate ("NADP"), catalyzes the conversion glucose-6-phosphate to 6-phosphogluconate and reduced NADP ("NADPH"). The resultant NADPH is coupled to the reduction of iodonitrotetrazolium ("INT"), or other reagent which when chemically reduced forms a colorimetrically detectable species, to form INT-formazan.

optionally, glucose may be measured, e.g., using glucose oxidase-catalyzed conversion of glucose to gluconic acid, thereby producing hydrogen peroxide, which may be detected colorimetrically by, for example, incubation with 4-aminoantipyrine and p-hydroxybenzene sulfonate or o-dianisidine in the presence of peroxidase to produce the colorimetrically detectable species quinoneimine dye or oxidized o-dianisidine, respectively. By comparison with a standard curve generated using known quantities of glucose, the amount of glucose in the sample can be determined.

The individual components required for the above-described glucose assays, as well as kits therefor, may be obtained from any commercial source, e.g., Sigma Chemical Co. (St. Louis, MO).

Insulin levels in biological samples may be determined by, for example, radioimmunoassay. Typically, an antibody against insulin is used that cross-reacts with proinsulin and split-proinsulin (Reaven et al. (1993) *J. Clin. Endo. Metab.* 76:44-48). A specific antibody is commercially available which only minimally (< 0.2%) cross-reacts with proinsulin and split-proinsulin (Human Insulin Specific RIA kit, Linco Research, Inc., St. Louis, MO). Assays employing either antibody may be used to assess the level of insulin in a biological sample. Preferably, a sample may be assayed with the cross-reacting antibody and with the specific antibody in order to determine the amounts of proinsulin present in the biological sample.

The solid oral carbohydrate diagnostic test meal disclosed herein contains a carbohydrate source providing an amount of starch, i.e., complex carbohydrate, which has been calibrated and standardized to provide a selected quantity of available carbohydrate upon ingestion of the test meal by a vertebrate subject. The preferred source of carbohydrate is oat starch formulated with a standardized quantity of β-glucan from oat grain, other cereal grain including, without limitation, any of the various cultivars of e.g., barley, oat, wheat, rye, corn, maize, sorghum and millet, or another source, such as potato, sweet potato, canna, arrowroot, tapioca (casava) sago, arum, triticale, rice, beans, peas, lentils; chestnuts, peanuts, inulin, lichen, or the like. Alternative, any synthetic starch well known in the art can be in included as a carbohydrate source. Other ingredients may also be included such as protein, fat, texture and palatability enhancers and the like. The test meal may be formulated to contain a physiologically balanced carbohydrate source, i.e., the test meal may contain the recommended diabetes dietary requirements of carbohydrate, fat and protein. Such dietary guidelines have been set forth by the American Diabetes Association ("ADA") (1994) *Diabetes Care* 17:519-522, the Canadian Diabetes Association ("CDA") (1989) *Beta Release* 13:8-17, and the European Association for the Study of Diabetes ("EASD") (1988) *Diab. Nutr. Metab.* 1;145-149, the disclosures of which are hereby incorporated in their entirety by reference. The CDA and EASD recommendations provide that carbohydrate should make up 50% or more of energy intake, fat intake should not exceed 30% of energy intake (of which it is recommended that 10% is saturated fat, 10% is monounsaturated fat and 10% is polyunsaturated fat), protein should be adjusted to meet individual energy requirements, e.g., about 12% to about 15% of energy intake, with the diet having high fiber soluble fiber content, e.g., 40 grams per day or 25 grams per 1000 kilocalories ("kcal"). Current ADA recommendations do not specify total amounts of dietary carbohydrate and fat, but recommend that these dietary components be individualized according to need. The ADA recommendations do provide that less than about 10% of energy intake should be from saturated fat and that about 60% to about 70% of energy intake should be from carbohydrate and monosaturated fat. Energy can be calculated in kcal and converted to kilojoules (kJ) by using the conversion factor of 1 kcal equal 4.18 kJ.

In one preferred formulation, the diagnostic test meal is formulated to contain about 45% to about 75%, preferably 50% to 65%, more preferably 55% to 60% of the energy provided in the meal from carbohydrates, preferably complex carbohydrates. In addition, the test meal may contain about 15% to 30% of energy from fat, low in saturated fat, e.g., less than about 10% to 30% of total fat energy, and higher in monounsaturated fat, e.g., greater than about 25% to 75%, preferably 45% to 55%, of total fat energy, with the remaining fat energy provided by polyunsaturated fat, about 10% to 25% energy from protein, with the optional addition of about 5 to 10 grams of dietary fiber.

Among the preferred constituents are fat obtained from vegetable oils, such as canola oil, rapeseed oil, flax oil, borage oil, safflower oil, soybean oil, coconut oil, evening-primrose oil, castor oil, olive oil, almond oil, peanut oil, linseed oil, corn oil and maize oil, fish oils such as cod liver oil or halibut oil, extracted oat fat or the like, protein obtained from soy flour, egg albumin, ovoglobulin, wheat gluten, whey, lactoglobulin, lactalbumin, meat and blood isolates, serum albumin, fish protein isolates, legume isolates, soy protein, quinoa protein, or the like, and dietary soluble fiber such as β-glucan in oat bran. In order to increase the palatability of the test meal, flavoring such as apple juice, cinnamon, lemon or orange extracts, or the like may be added to the test meal. Other standard food additives may be incorporated into the test meal, for example, acidulants, anticaking agents, antimicrobial agents, antioxidants, baking aids, bleaching agents, buffering agents, bulking agents, carmelization aids, carriers, clarifying agents, clouding agents, colorants, color fixatives, cooking media, defoamers, dough conditioners, emulsifiers, enzymes, filter aids, firming agents, flavor enhancers, flavors, foam stabilizers, food starch modifiers, gums, humectants, hydrocolloids, leavening agents, lubricants, masticatory substances, maturing agents, microorganisms, neutralizers, nonnutritive sweeteners, nutrients and dietary supplements, preservatives, propellants, protein sources, refrigerants, salt substitutes, sequestrants, solvents, stabilizers, surface-active agents, surface-finishing agents, sweeteners, texturizers, thickeners, vitamins, and the like. *See, e.g*., Furia (1972 and 1980) *Handbook of Food Additives*, 2d ed., Volumes I and II, CRC Press Inc. (West Palm Beach, FL).

In one preferred embodiment, a 90.5-gram solid test meal is provided in the form of a bar. The test meal, having 326 kcal of energy provided by the meal, consists of 50.0 grams of carbohydrate (61.5% of the meal energy) derived from starch extracted from oat groats, 8.9 grams of fat (24.5% of the meal energy) derived from canola oil and extracted oat fat, 11.4 grams of protein (14.0% of the meal energy) derived from soy flour, egg albumin and/or wheat gluten, 6.9 grams of fiber derived from β-glucan in oat bran, and apple juice, cinnamon and orange juice for flavoring.

A particularly preferred formulation for a 133.25-gram (wet weight) test meal in the form of a bar includes the following ingredients (in grams): water (44), rolled oat flakes (19.82), pin-milled flour (available as Ostar® PM flour from Canamino, Inc., Saskatoon, Saskatchewan, Canada) ((13.21), pin-milled starch (available as Ostar® PM starch from Canamino, Inc.) (13.21), oat bran (6.6), liquid honey (9.55), canola oil (7.78), soy protein (5.73), sugar (3.69), glycerin (3.34), gluten (2.86), baking powder (2.192), cinnamon (1.096), salt (0.116), and allspice (0.041).

The amount of available carbohydrate in the test meal may be calibrated to achieve a selected glycemic response or, preferably, a selected glycemic index, determined as described above. A reference glycemic response may be obtained using, for example, an oral liquid glucose standard containing a quantity of glucose, for example, 25 grams, 50 grams, 75 grams or 100 grams of glucose. The amount of available carbohydrate in a test meal may be standardized to achieve a selected 30-minute, 45-minute, 60-minute, 90-minute 120-minute, or the like, postprandial blood glucose response. For a test meal calibrated against an oral liquid glucose reference, it is preferred that the reference glycemic response be determined using a 75-gram oral liquid glucose beverage, e.g., Glucodex®. For a test meal calibrated to achieve a selected glycemic index, a preferred reference glycemic index may obtained using a 50-gram carbohydrate portion of white bread. Thus, for example, the glycemic response or glycemic index obtained for a subject after ingestion of the test meal may be compared to the precalibrated glycemic index or glycemic response of the test meal.

The carbohydrate source can be obtained commercially, e.g., Canamino (Saskatoon, Saskatchewan, Canada). Optionally and preferably, the carbohydrate source may be prepared from cereal grains, and preferably oats, using the method disclosed in U.S. Patent No. 4,435,429, issued to Burrows et al.. Briefly, the process involves separation of endospermic tissue from other tissues in grain by first soaking the grain in an aqueous medium, pH 3-7, at 40°C to 70°C until the grain has absorbed at least its own weight of the aqueous medium and the endosperm portion of the grain has been liquified by the action of enzymes indigenous to the cell wall. The grain is then split under pressure to release virtually all the liquified endosperm. The endosperm can then be separated from the other tissues of the grain. The grain can be whole, dehulled or hulless. The aqueous medium preferably contains up to about 0.1 wt.% SO₂. Using this method, low fiber, off-white flour can be produced in the wet or dry state ready for further fractionating.

Optionally, and in most cases preferably, the carbohydrate source from cereal grains, preferably oat grains, may be prepared using the method described in U.S. Patent No. 5,169,660, issued to Collins et al., which is hereby incorporated in its entirety by reference. The method involves first steeping the cereal grain in water for a period of time sufficient to substantially completely liquify the endosperm. The steeped grain is then macerated in an aqueous ethanol solution to liberate the liquid endosperm. Insoluble bran is then separated and recovered from the aqueous ethanol solution and the insoluble flour is separated and recovered from the bran-free aqueous ethanol solution. Recovery of the insoluble bran and flour from the aqueous ethanol solution may be effected by passing the solution over sequentially finer mesh screens. This method is particularly useful for producing relatively pure bran and flour from oat, wheat and rye grains.

The test meal may be in any solid physical form such as a bar, wafer, biscuit, cookie or the like. Preferably, the meal is in the form of a bar. More preferably, the bar is scored to provide two or more portions that may be broken off and ingested if it is desired that the subject ingest a lower quantity, if multiple tests are to be administered or if the subject is a juvenile.

A test meal bar may be made by routine methods well known to those of skill in the art. For example, the components of the test meal may be blended in predetermined proportions with a binding agent, if necessary, selected so as to preserve the component proportions of the bar, e.g., gelatin, β-glucan or wheat gluten, to form a mixture. The mixture may be molded, pressed, poured, extruded or otherwise formed into a desired shape. The shaped bar may also be baked, steamed, dried or otherwise processed, as required, to set the shape of the bar. *See, e*.*g*., U.S. Patent No. 5,200,215 to Slade et al.

The test meal disclosed herein produces a more precise (CV ≈ 3%-5%) and more physiological measurement of postprandial glucose and insulin responses compared to liquid glucose beverages such as Glucodex®. Furthermore, the solid test meal produces more precise responses than liquid meals such as Enrich® (Ross Laboratories, Montreal, Canada). Thus, in using the solid test meal, it is possible to predict blood glucose or insulin responses with only 3%-5% error. Accordingly, the solid test meal can be used as a substitute for the liquid 75-gram liquid glucose standard for the diagnosis and/or management of diabetes mellitus, insulin resistance, impaired glucose tolerance, maturity onset diabetes or gestational diabetes, insulin resistance or for the regulation of hypoglycemia. In addition, the test meal can be used to determine postprandial glucose and/or insulin responses.

Conventional fasting blood glucose or insulin determinations are unreliable for the assessment of disorders of carbohydrate metabolism because as many as 39% of the subjects in the upper range of normal fasted glucose levels show diabetic postprandial glucose responses. Thus, they can only be found with a 60-minute or greater postprandial carbohydrate challenge. Using the novel diagnostic solid test meal disclosed herein, a postprandial glucose response assessment can be initiated one or two hours before clinical laboratory examination, e.g., a subject ingests the test meal one or two hours before blood is drawn at the clinic. In addition, the test meal can be used for self-diagnosis, self-teaching and blood glucose self-monitoring.

Insulin measurements require the ingestion of carbohydrate, fat and protein to be accurate; the OGTT cannot be used to measure postprandial plasma insulin levels accurately. One method for determining postprandial insulin responses using the solid test meal involves first determining a subject's fasting insulin blood levels using methods well known in the art, for example, radioimmunoassay. Postprandial insulin levels are then determined 60, 90 and/or 120 minutes after the ingestion of a diagnostic test meal and compared with predetermined and recognized standards (*see, e.g*., Reaven et al. (1993) *Diabetes* 42:1324 and DeFronzo (1988) *Diabetes* 37:667) or compared with previous tests on the individual subject.

In addition, the diagnostic test meal can be used in the management of disorders of carbohydrate metabolism. In particular, the test meal can be used as a standard test meal to titrate drug dosage for the treatment of these diseases in individual subjects with oral antidiabetic agents. For example, α-glucosidase inhibitors, e.g., acarbose, are a new class of drugs which improve blood glucose control in diabetics (Clissold et al. (1988) *Drugs* 35:214-243; Chiasson et al. (1994) *Ann. Int. Med.* 121:928-935). Following a liquid test meal, acarbose treatment causes a decrease in the mean glucose peak determined 90 minutes postprandial in patients with NIDDM (Chiasson et al., *supra*). However, flatulence, abdominal distension and diarrhea are major side-effects associated with α-glucosidase inhibitors. This, it is desirable to titrate the dose of acarbose to that dose required to suppress postprandial plasma glucose.

The diagnostic test meal disclosed herein also affords a method by which such dosing regimens can be titrated to an optimal level. In order to assess the dosage of drugs required to decrease postprandial blood glucose a predetermined amount, a solid test meal may be administered to a subject and plasma glucose levels monitored for 0 (i.e., the 10-to 16-hour fasting level) 30, 45, 60, 90 and 120 minutes. The incremental area under the glycemic response curve is calculated geometrically, ignoring area beneath the fasting value (Wolever et al. (1991) *Am. J. Clin. Nutr.* 54:846-854). This procedure is repeated after administration of the antidiabetic agent to determine if the drug produces the desired diminution in glycemic response. The dose of drug administered is then adjusted upward or downward as necessary to achieve the desired effect. The process may be repeated as often as required until the desired drug response is achieved. In addition, the efficacy of the treatment may be assessed periodically and the drug dosage adjusted as necessary.

A determination of the efficacy of an antidiabetic drug, and whether adjustment of the drug dosage is required, may be made by monitoring changes in the postprandial blood glucose concentration following administration of the drug. Acceptable limits of a drug-induced change in postprandial blood glucose concentration as indicating either acceptable efficacy or a necessity for further titration of the dosage are well known to those of skill in the art. For example, it is generally accepted that, in a subject that has been treated with a first dose of an antidiabetic agent, a second higher or lower dose of the agent that respectively effects a decrease or increase in blood glucose of approximately 2 mmol/L is considered equally efficacious.

An additional use for the solid diagnostic test meal is in the area of self-diagnosis and self-monitoring. Using currently available techniques well known to those in the art, a subject can obtain fasting and postprandial blood samples which can be analyzed for glucose levels using, for example, the One Touch® II blood glucose monitoring system (Lifescan, Inc., Milpitas, California). Optionally, urinary glucose levels may be assessed using the Tes-Tape® glucose enzymatic test strip (Eli Lilly, Indianapolis, Indiana).

Furthermore, as the invention represents a nutritionally balanced meal it can be used in the treatment of hypoglycemia and diabetic shock.

It is to be understood that while the invention has been described in conjunction with the preferred specific embodiments thereof, that the description above as well as the examples which follow are intended to illustrate and not limit the scope of the invention. Other aspects, advantages and modifications within the scope of the invention will be apparent to those skilled in the art to which the invention pertains.

The following examples are put forth so as to provide those of ordinary skill in the art with a complete disclosure and description of how to make and use the compounds of the invention, and are not intended to limit the scope of what the inventors regard as their invention. Efforts have been made to insure accuracy with respect to numbers (e.g., amounts, temperature, etc.) but some errors and deviations should be accounted for. Unless indicated otherwise, parts are parts by weight, temperature is in °C and pressure is at or near atmospheric.

### B. Experimental

### Materials and Methods

Finger-prick capillary blood samples (≈ 200 µl) were collected into fluoro-oxalate tubes and stored at -20°C for a maximum of 24 hours. Whole blood glucose was measured using a model 2300 STAT glucose analyzer (Yellow Springs Instruments, Ohio). Incremental areas under the glycemic response curves ("AUC") were calculated geometrically, ignoring area beneath the fasting value (Wolever et al. (1991) *Am. J. Clin. Nutr.* 54:846-854).

The mean and standard deviation ("SD") of the results for three repeats of each test meal (unless otherwise indicated) were calculated for each subject and the coefficient of variation ("CV") was calculated by expressing the SD as a percent of the mean. Where appropriate, analysis of variance was performed with subject and test meal as the variables, and the Neuman-Kuels procedure used to adjust for multiple comparisons.

Plasma insulin was measured by radioimmunoassay. Normally, proinsulin is almost completely cleaved when insulin is secreted. Accordingly, in normal subjects proinsulin is present only in trace amounts in plasma. However, in subjects with IGT and diabetes, insulin secretion is abnormal and larger amounts of proinsulin are present in plasma. Since proinsulin cross-reacts with the antibody in the conventional assay, this may yield results that indicate falsely elevated insulin levels. Although the amount of proinsulin present in plasma of hyperinsulinemic subjects is not enough to account for the hyperinsulinemia, i.e., the true insulin level is elevated (Reaven et al., *supra*), both assays are used for assessing the insulin responses described in the Examples below.

Plasma glucose was measured using the hexokinase method (Bergmeyer et al. (1974) in Bergmeyer et al., eds. *Methods of Enzymatic Analysis,* Academic Press (New York).

All procedures involving human subjects were approved by the University of Toronto Human Subjects Review Committee or other appropriate review committee.

### EXAMPLE 1

### Determination of Glycemic Index of Test Meal Components

The purpose of this experiment was to determine the glycemic index of oat starch, oat flour and oat bran, which are the main ingredients for the test meal.

Seven normal subjects were studied on six different occasions in the morning after overnight fasts. They ate 50-gram available carbohydrate portions of oat starch (56 gm), oat flour (64 gm), oat bran (105 gm) and white bread (100 gm). The white bread ingestion was repeated three times by each subject for glycemic index calculations. The oat products were prepared by adding 250 ml boiling water to the preweighed product, stirring well and letting stand for three to four minutes. Each test meal was consumed with a 250 ml to 500 ml drink of the subject's choice of water, coffee or tea (with 30 ml 2% milk if desired); the drink was standardized for each subject. Finger-prick capillary blood test samples were obtained from the fasting subjects prior to ingestion of the meal and at 15, 30, 45, 60, 90 and 120 minutes after starting to eat the test meal. Test meals were consumed by each subject within 15 minutes. Glycemic index was calculated as the AUC for each oat product expressed as a percent of the mean AUC for the three white bread meals ingested by each subject.

The mean coefficient of variation ("CV") for the seven subjects for fasting blood glucose levels and for blood glucose levels 60 and 120 minutes after white bread ingestion were 2.7%, 3.7% and 5.3%, respectively. The mean CV for the glycemic index was 11.9%. This variability is considerably less than the mean CV of 22% expected based on previous data for normal subjects taking repeated tests of white bread meals.

The glycemic indices (expressed as the mean ± S.E.M.) of oat starch (124 ± 12) and oat flour (127 ± 19) were virtually identical and not significantly different from that of white bread (100). The peak glycemic responses of oat starch and oat flour were significantly greater than that of white bread. The glycemic index of oat bran (54 ± 10) was significantly less than that of white bread, as was its peak glycemic response.

### EXAMPLE 2

### Comparison of Reproducibility of the Glycemic Responses to Oral Glucose Tolerance Tests and Carbohydrate Diagnostic Test Meals

Ten normal subjects (4 male, 6 female; age: 33 ± 3 years; body mass index ("BMI"): 23.8 ± 1.1 kg/m²) were studied after 10-12 hour overnight fasts on nine separate mornings over a six-week period. One subject, found to have impaired glucose tolerance during the study, had had a normal OGTT six months prior to the study. Subjects consumed either 75 grams of glucose in 300 ml orange-flavored water (Glucodex®) or a 50-gram carbohydrate portion of white bread or a solid oral carbohydrate diagnostic test meal consisting of an oat bar.

White bread was baked in 250-gram carbohydrate loaves containing 334 grams of all purpose flour (Maple Leaf Mills, Toronto, Ontario), 7 grams of sucrose, 6 grams of yeast, 4 grams of salt and 2500 mL water. Each loaf was cut into 50-gram portions (discarding crust ends).

The oat bar contained bran, starch and flour refined from oats under highly standardized conditions. The 50-gram carbohydrate portion of oat bar weighed 104 grams and contained 352 kcal, 11.7 grams fat, 11.8 grams protein, 42.2 grams starch, 7.8 grams sugars and 5.9 grams dietary fiber. The energy content expressed in kcal can be calculated based on the energy content per gram of fat (9 kcal), protein (4 kcal), available carbohydrate (3.8 kcal), respectively.

The glucose solution was taken with 250 ml water, and bread portions and oat bars were taken with 450 ml water. Subjects repeated each test meal three times and test meals were consumed within 10 minutes.

Finger-prick capillary blood samples were collected prior to and 15, 30, 45, 60, 90 and 120 minutes after starting to eat and whole blood glucose was analyzed as described above. Incremental areas under the glycemic response curves were calculated by the method described above.

The mean fasting blood glucose concentration was similar before each of the three test meals. After glucose ingestion, mean blood glucose was significantly greater than after bread and oat bar at every time point; no significant differences were observed in mean blood glucose between bread and oat bar at any time point. The incremental area under the curve after glucose (284 ± 42 mmol·min/L) was greater than after bread (146 ± 22 mmol·min/L) (p < 0.01) and oat bar (124 ± 19 mmol·min/L) (p < 0.01), with no significant difference between bread and oat bar.

The within-subject CV of blood glucose for the three repeats of oral glucose were greater than for the three bread tests and at 15, 30 and 90 minute (p < 0.05). The CVs for oat bar were not significantly different from glucose or bread, except that at 120 minutes both oat bar and bread CVs were less than after oral glucose (p < 0.01). For seven of the ten subjects, the CV of the 120-minute blood glucose after 75 grams glucose was > 7%, while after bread, seven of the ten subjects had CV < 7%. The mean CV of the areas under the curve was less for bread than oat bar (p < 0.05), but the area CVs for bread and oat bar were not significantly different from that for glucose. The difference between subjects for blood glucose CV was significant at 120 minutes (F_{(9,18)}=2.72) but not at any other time. There was a positive relationship between the mean 120-minute blood glucose concentration and the CV of the 120-minute blood glucose.

The mean within-individual CV of the 2-hour postprandial blood glucose was 12.9% in subjects who had been given 75 grams of glucose. In contrast, the mean within-individual CV of the 2-hour postprandial blood glucose was about 5% for bread or the test meal.

### EXAMPLE 3

### Solid Oral Carbohydrate Diagnostic Test Meal

A typical analysis of a 90.5-gram and a 98.0-gram solid oral carbohydrate test meal is given in Table I below.

**TABLE I**

| COMPOSITIONAL ANALYSIS OF A SOLID TEST MEAL | | | |
|---|---|---|---|
| | A | B | Source |
| Meal Weight | 90.5 grams | 98.0 grams | |
| Caloric Content | 326 Kcal | 350 Kcal | |
| Fat | 8.9 grams (24.5% energy) | 11.3 grams | canola oil and extracted oat fat |
| Protein | 11.4 grams (14.0% energy) | 12.2 grams | soy flour and/or egg albumin and wheat gluten |
| Carbohydrate | 50.0 grams^{a} (61.5% energy) | 50 grams (40.5^{a} + 9.5^{b}) | ^{a} oat starch* ^{b} sugars |
| Fiber | 6.9% | 5.9 grams | β-Glucan** in oat bran |
| Flavoring | | | apple juice, cinnamon, orange |

| | | | |
|---|---|---|---|
| * Starch extracted from oat groats. 80% of the available carbohydrate is from isolated modified oat starch. | | | |
| ** β-Glucan 19% in oat bran. | | | |

### EXAMPLE 4

### Evaluation of the Effect of Acarbose on Glucose Tolerance, Hyperinsulinemia and Insulin Sensitivity

The purpose of this experiment was to evaluate the effect of acarbose, and α-glucosidase inhibitor, on glucose tolerance, hyperinsulinemia and insulin sensitivity in subjects with impaired glucose tolerance. Subjects were randomly treated in a double-blind fashion with placebo (n = 12) or acarbose (n = 11), 100 mg three times a day ("TID") for four months. Each subject was tested as follows before and after treatment.

The subjects were fed a liquid test meal followed by measurements of plasma glucose, insulin and proinsulin 30, 60, 90, and 120 minutes postprandial. In addition, an insulin suppression test was done on each subject before and after treatment using the steady state plasma glucose ("SSPG") as an index of insulin sensitivity. Thus, subjects were continuously infused with insulin, somatostatin and glucose for three hours. Plasma glucose levels were taken at 120 minutes, 150 minutes and 180 minutes. The means of these levels (the SSPG) was used as a measure of insulin sensitivity.

The mean peak plasma glucose response to the liquid test meal at 60 minutes postprandial was 8.4 ± 0.6 mmol/L before randomization. The respective responses after placebo and acarbose treatment were 8.2 ± 0.9 mmol/L and 6.3 ± 0.3 mmol/L (p < 0.0001). The mean peak proinsulin response at 120 minutes postprandial was 76.8 pmol/L before randomization. After placebo and acarbose treatment the respective responses were 81.1 pmol/L and 58.2 pmol/L (p < 0.001).

The mean SPPG was 13.3 ± 1.0 mmol/L before randomization. After placebo and acarbose treatments, the respective mean SPPGs were 13.8 ± 0.4 and 10.0 ± 1.2 mmol/L (p < 0.032).

These data indicate that, in subjects with IGT, acarbose treatment induced an improvement in glucose tolerance, a decrease in insulin levels and a decrease in insulin resistance.

### EXAMPLE 5

### Comparison of the Relationship Between Plasma Glucose and Insulin Responses to Oral Glucose and the Test Meal in Obese Subjects, IGT Subjects and Subjects with Type 2 Diabetes

Four groups of adult (≥ 18 years of age) were studied with 10 volunteers in each group: nonobese (BMI < 27 kg/m²) normal subjects; obese (BMI ≥ 27 kg/m²) normal subjects; subjects with impaired glucose tolerance ("IGT") within the last twelve months; and subjects with non-insulin dependent (type 2) diabetes treated by diet alone.

Each subject was studied on eight occasions after a 12-hour overnight fast. The subjects consumed either 75 grams of glucose in 300 ml orange-flavored water (Glucodex®) ("GTT") or 50 grams of available carbohydrate in the form of a 98.0-gram test meal in the form of a bar ("TMB") as described in Table I. The test meals were administered to each subject in four consecutive blocks with each block consisting of one GTT and one TMB. The order of tests within each block was randomized, with randomization stratified according to group and gender.

Both venous and capillary blood samples were obtained at each time point. The capillary blood sample was taken from a finger prick immediately after the venous sample had been obtained by way of an intravenous cannula. After-fasting samples of venous and capillary blood were obtained, the subject consumed GTT or TMB over a ten-minute period. Additional blood samples were taken at 15, 30, 45, 60, 90 and 120 minutes after the start of the test meal (t₀ = start of test meal consumption).

Reproducibility of the repeated tests in each group of subjects is expressed as the coefficient of variance which is calculated from the 4 repeated tests done by each subject. Reproducibility is determined for the 60-minute and 120-minute time points and for the incremental area under the curve. The differences between plasma glucose and insulin responses of the different test meals (GTT and TMB) are determined primarily using the area under the curve and secondarily using each time point with analysis of variance of the repeated measurements.

Within-subject variability of blood glucose and insulin responses following ingestion of 75 grams glucose are compared with the reproducibility after ingestion of 50 grams of available carbohydrate in test bar format. In addition, the relationship between the plasma glucose and insulin responses after oral glucose and the test bar meal are compared in nonobese and obese normal subjects, subjects with impaired glucose tolerance and subjects with type 2 diabetes treated by diet alone.

To date 44 GTT tests and 41 TMB tests have been completed. Six subjects have completed the study, and 16 subjects (8 normal, 5 IGT, and 3 diabetic) have taken at least 1 glucose and one bar test. The mean 2-hour postprandial plasma glucose of these 16 subjects after the GTT varied from 3.6 mmol/L to 19.9 mmol/L. The mean 2-hour postprandial glucose after oral glucose was linearly related to the 2-hour postprandial glucose after the test bar (r = 0.95; r² = 0.90; p < 0.001). The data show that a 2-hour postprandial plasma glucose of ≥ 8.7 mmol/L after the TMB corresponds to a 2-hour postprandial plasma glucose of ≥ 11.1 mmol/L after GTT (i.e., diabetes), and that a 2-hour postprandial plasma glucose of ≤ 6.3 mmol/L after the TMB corresponds to ≤ 7.7 mmol/L after GTT (i.e., normal). These results suggest that the test bar will be useful as a valid screening test for diabetes.

### EXAMPLE 6

### Effect of Acarbose on the Postprandial Blood Glucose Response to Ensure® and the Solid Test Meal

Ensure®, a liquid formula diet, has been used as a standard test meal in many clinical trials of acarbose in the treatment of diabetes. However, the type of carbohydrate in Ensure® differs markedly from that of a normal diet; starch contributes 25-30% of energy in a normal diet, but Ensure® contains no starch. Thus, the pattern of postprandial blood glucose response after Ensure® may differ from that of a normal diet. In addition, acarbose may have a different effect on postprandial blood glucose after consumption of Ensure® than after starchy foods. In order to compare the postpradial blood glucose response, and the effect of acarbose thereon, to white bread described in Example 2, Ensure® and the 98.0-gram solid test meal in the form of a bar ("TMB") as described in Table I, we studied 2 groups of normal subjects on separate mornings after an overnight fast. On each day, subjects ate a test meal containing 50 grams of carbohydrate (total carbohydrate minus total dietary fiber). After-fasting finger-prick blood samples for glucose analysis were obtained. Additional blood samples were taken at 15, 30, 45, 60, 90 and 120 minutes after the start of the test meal (t₀ = start of test meal consumption).

In one study, 9 subjects (5 female, 4 male, age 31 ± 2 years, BMI 22.9 ± 1.4 kg/m²) tested white bread, TMB or Ensure®. Compared to white bread, postprandial blood glucose after Ensure® tended to be higher at 15 minutes (p = 0.07) and was significantly lower at 45 (p = 0.003), 60 (p = 0.012), 90 (p = 0.025) and 120 (p = 0.007) minutes. The incremental area under the curve (AUC) after Ensure®, 83 ± 12 mmol-min/L was 68 ± 8% that of white bread, 125 ± 15 mmol-min/L (p = 007). Blood glucose after the TMB was not significantly different from that after white bread at any time point, and the AUC after TMB, 110 ± 16 mmol-min/L, was similar to that after white bread. The mean AUC after Ensure® was 75% that after the TMB.

In another study, 6 subjects (2 female, 4 male, age 34 ± 4y, BMI 23.9 ± 1.4kg/m2) tested Ensure® or TMB with 50 mg acarbose or placebo using a randomized latin square design. The results of this study are shown in FIGS. 1, 2, and 3. The mean AUC after Ensure® plus placebo, 88 ± 11 mmol-min/L, was 73% of that after the TMB, 120 ± 16 mmol-min/L. The AUC after Ensure® plus acarbose, 56 ± 18 mmol-min/L, tended to be less than after Ensure® plus placebo, but the difference was not significant. The AUC after TMB plus acarbose, 78 ± 8 mmol-min/L, was significantly less than after TMB plus placebo. Acarbose reduced the AUC after Ensure® by 35 ± 20% (not significant), and after TMB by 30 ± 11% (p < 0.05). It is concluded the blood glucose response of the TMB is more representative of a normal diet than Ensure®. In normal subjects, acarbose reduces postprandial blood glucose to a similar extent after Ensure® and the TMB, but the effect may be more consistent after the TMB.

## Claims

1. A use of a solid oral diagnostic test meal for determining the postprandial concentration of a blood constituent in a vertebrate subject, said diagnostic test meal comprising:
a) a complex carbohydrate that provides a quantity of available carbohydrate effective to increase blood glucose levels in a vertebrate subject when ingested by the subject, wherein the carbohydrate is present in an amount in the range of about 50% to about 65% of the energy provided in the meal;
b) a source of dietary fat, wherein the dietary fat is present in an amount in the range of about 15% to about 30% of the energy provided in the meal;
c) a source of dietary protein, wherein the dietary protein is present in an amount in the range of about 10% to about 25% of the energy provided in the meal; and, optionally,
d) a source of dietary fiber and flavoring.

2. The use according to claim 1, wherein the complex carbohydrate is derived from a cereal grain selected from the group consisting of barley, oat, wheat, rye, corn, maize, sorghum and millet.

3. The use according to claim 2, wherein the cereal grain is oat.

4. The use according to any of claims 1-3, wherein the meal is in the form of a bar.

5. The use according to any of claims 1-4, wherein the test meal is used for diagnosing a disorder of carbohydrate metabolism.

6. The use according to claim 5, wherein the test meal is used for diagnosing a disorder of carbohydrate metabolism selected from the group consisting of diabetes mellitus, impaired glucose tolerance, insulin resistance, non-insulin dependent diabetes, maturity onset diabetes, gestational diabetes and hyperinsulinemia.

7. The use according to any of claims 1-6, wherein the blood constituent is glucose.

8. The use according to any of claims 1-6, wherein the blood constituent is insulin.

9. The use according to any of claims 1-4, wherein the test meal is used for managing the dosage of a drug that decreases postprandial glucose concentration.

10. The use according to claim 9, wherein the drug is insulin.

11. The use according to any of claims 1-4, wherein the test meal is used for self-monitoring and/or self-diagnosis of diabetes.

12. A use of a kit for determining the postprandial concentration of a blood constituent in a vertebrate subject comprising a solid oral diagnostic test meal comprising:
a) a complex carbohydrate that provides a quantity of available carbohydrate effective to increase blood glucose levels in a vertebrate subject when ingested by the subject, wherein the carbohydrate is present in an amount in the range of about 50 % to about 65 % of the energy provided in the meal;
b) a source of dietary fat, wherein the dietary fat is present in an amount in the range of about 15% to about 30% of the energy provided in the meal;
c) a source of dietary protein, wherein the dietary protein is present in an amount in the range of about 10 % to about 25 % of the energy provided in the meal; and, optionally,
d) a source of dietary fiber and flavouring.

13. The use according to claim 12, wherein the kit is used for diagnosing a disorder of carbohydrate metabolism.

14. The use according to claim 13, wherein the kit is used for diagnosing a disorder of carbohydrate metabolism selected from the group consisting of diabetes mellitus, impaired glucose tolerance, insulin resistance, non-insulin dependent diabetes, maturity onset diabetes, gestational diabetes and hyperinsulinemia.

15. The use according to claim 12, wherein the blood constituent is glucose.

16. The use according to claim 12, wherein the blood constituent is insulin.

17. The use according to claim 12, wherein the kit is used for managing the dosage of a drug that decreases postprandial glucose concentration.

18. The use according to claim 16, wherein the drug is insulin.

19. The use according to claim 12, wherein the kit is used for self-monitoring and/or self-diagnosis of diabetes.

## Patentansprüche

1. Verwendung einer festen oralen diagnostischen Testmahlzeit zum Bestimmen der postprandialen Konzentration eines Blutbestandteils in einem Wirbeltierprobanden, wobei die diagnostische Testmahlzeit umfaßt:
a) ein komplexes Kohlenhydrat, das eine Menge eines verfügbaren Kohlenhydrats bereitstellt, das eine Erhöhung der Blutglucosekonzentration in einem Wirbeltierprobanden bewirkt, wenn es von dem Probanden eingenommen wird, wobei das Kohlenhydrat in einer Menge im Bereich von etwa 50 % bis etwa 65 % der in der Mahlzeit zur Verfügung gestellten Energie vorliegt;
b) eine Quelle eines Diätfetts, wobei das Diätfett in einer Menge im Bereich von etwa 15 % bis etwa 30 % der mit der Mahlzeit bereitgestellten Energie vorliegt;
c) eine Quelle eines Diätproteins, wobei das Diätprotein in einer Menge im Bereich von etwa 10 % bis etwa 25 % der in der Mahlzeit bereitgestellten Energie vorliegt; und gegebenenfalls
d) eine Quelle einer Diätfaser und eines Geschmacksmittels.

2. Verwendung nach Anspruch 1, wobei das komplexe Kohlenhydrat von einem Getreidekorn stammt, das ausgewählt ist aus der Gruppe bestehend aus Gerste, Hafer, Weizen, Roggen, Korn, Mais, Sorghum und Hirse.

3. Verwendung nach Anspruch 2, wobei das Getreidekom Hafer ist.

4. Verwendung nach einem der Ansprüche 1-3, wobei die Mahlzeit in Form eines Riegels vorliegt.

5. Verwendung nach einem der Ansprüche 1-4, wobei die Testmahlzeit zur Diagnose einer Störung des Kohlenhydratstoffwechsels verwendet wird.

6. Verwendung nach Anspruch 5, wobei die Testmahlzeit zur Diagnose einer Störung des Kohlenhydratstoffwechsels verwendet wird, die ausgewählt ist aus der Gruppe bestehend aus Diabetes mellitus, gestörte Glucosetoleranz, Insulinresistenz, nicht-insulinabhängigem Diabetes, im Erwachsenenalter eintretendem Diabetes, Gestationsdiabetes und Hyperinsulinämie.

7. Verwendung nach einem der Ansprüche 1-6, wobei der Blutbestandteil Glucose ist.

8. Verwendung nach einem der Ansprüche 1-6, wobei der Blutbestandteil Insulin ist.

9. Verwendung nach einem der Ansprüche 1-4, wobei die Testmahlzeit zum Steuern der Dosierung eines Arzneistoffs verwendet wird, der die postprandiale Glucosekonzentration senkt.

10. Verwendung nach Anspruch 9, wobei der Arzneistoff Insulin ist.

11. Verwendung nach einem der Ansprüche 1-4, wobei die Testmahlzeit zur Eigenüberwachung und/oder Eigendiagnose von Diabetes verwendet wird.

12. Verwendung eines Kits zum Bestimmen der postprandialen Konzentration eines Blutbestandteils in einem Wirbeltierprobanden, umfassend eine feste orale diagnostische Testmahlzeit, die umfaßt:
a) ein komplexes Kohlenhydrat, das eine Menge eines verfügbaren Kohlenhydrates bereitstellt, das einen Anstieg der Blutglucosekonzentration in einem Wirbeltierprobanden bewirkt, wenn es von dem Probanden eingenommen wird, wobei das Kohlenhydrat in einer Menge im Bereich von etwa 50 % bis etwa 65 % der in der Mahlzeit bereitgestellten Energie vorliegt;
b) eine Quelle eines Diätfetts, wobei das Diätfett in einer Menge im Bereich von etwa 15 % bis etwa 30 % der mit der Mahlzeit bereitgestellten Energie vorliegt;
c) eine Quelle eines Diätproteins, wobei das Diätprotein in einer Menge im Bereich von etwa 10 % bis etwa 25 % der in der Mahlzeit bereitgestellten Energie vorliegt; und gegebenenfalls
d) eine Quelle einer Diätfaser und eines Geschmacksmittels.

13. Verwendung nach Anspruch 12, wobei der Kit zur Diagnose einer Störung des Kohlenhydratstoffwechsels verwendet wird.

14. Verwendung nach Anspruch 13, wobei der Kit zur Diagnose einer Störung des Kohlenhydratstoffwechsels verwendet wird, die ausgewählt ist aus der Gruppe bestehend aus Diabetes mellitus, gestörte Glucosetoleranz, Insulinresistenz, nicht-insulinabhängiger Diabetes, mit Erwachsenenalter eintretender Diabetes, Gestationsdiabetes und Hyperinsulinämie.

15. Verwendung nach Anspruch 12, wobei der Blutbestandteil Glucose ist.

16. Verwendung nach Anspruch 12, wobei der Blutbestandteil Insulin ist.

17. Verwendung nach Anspruch 12, wobei der Kit zum Steuern der Dosierung eines Arzneistoffs verwendet wird, der die postprandiale Glucosekonzentration absenkt.

18. Verwendung nach Anspruch 16, wobei der Arzneistoff Insulin ist.

19. Verwendung nach Anspruch 12, wobei der Kit zur Eigenüberwachung und/oder Eigendiagnose von Diabetes verwendet wird.

## Revendications

1. Utilisation d'un repas test diagnostique oral solide pour déterminer la concentration post-prandiale d'un constituant du sang chez un sujet vertébré, ledit repas test diagnostique comprenant :
a) un glucide complexe qui fournit une quantité de glucide disponible efficace pour augmenter les teneurs en glucose sanguin chez un sujet vertébré lorsqu'il est ingéré par le sujet, le glucide étant présent en une quantité dans l'intervalle d'environ 50 % à environ 65 % de l'énergie fournie dans le repas ;
b) une source de graisse alimentaire, la graisse alimentaire étant présente en une quantité dans l'intervalle allant d'environ 15 % à environ 30 % de l'énergie fournie dans le repas ;
c) une source de protéine alimentaire, la protéine alimentaire étant présente en une quantité dans l'intervalle d'environ 10 % à environ 25 % de l'énergie fournie dans le repas ; et éventuellement
d) une source de fibre alimentaire et d'agent aromatisant.

2. Utilisation selon la revendication 1, dans laquelle le glucide complexe est dérivé d'une graine de céréales choisie dans le groupe consistant en orge, avoine, blé, seigle, maïs, sorgho et millet.

3. Utilisation selon la revendication 2, dans laquelle la graine de céréale est de l'avoine.

4. Utilisation selon l'une quelconque des revendications 1-3, dans laquelle le repas est sous la forme d'une barre.

5. Utilisation selon l'une quelconque des revendications 1-4, dans laquelle le repas test est utilisé pour diagnostiquer un trouble du métabolisme des glucides.

6. Utilisation selon la revendication 5, dans laquelle le repas test est utilisé pour diagnostiquer un trouble du métabolisme des glucides choisi dans le groupe consistant en diabète sucré, tolérance au glucose altérée, résistance à l'insuline, diabète non insulino-dépendant, diabète de l'adulte, diabète gestationnel et hyperinsulinémie.

7. Utilisation selon l'une quelconque des revendications 1-6, dans laquelle le constituant du sang est le glucose.

8. Utilisation selon l'une quelconque des revendications 1-6, dans laquelle le constituant du sang est l'insuline.

9. Utilisation selon l'une quelconque des revendications 1-4, dans laquelle le repas test est utilisé pour ajuster la posologie d'un médicament qui réduit la concentration de glucose post-prandiale.

10. Utilisation selon la revendication 9, dans laquelle le médicament est l'insuline.

11. Utilisation selon l'une quelconque des revendications 1-4, dans laquelle le repas test est utilisé pour l'autocontrôle et/ou l'autodiagnostic du diabète.

12. Utilisation d'un kit pour déterminer la concentration post-prandiale d'un constituant du sang chez un sujet vertébré, comprenant un repas test diagnostique oral solide comprenant :
a) un glucide complexe qui fournit une quantité de glucide disponible efficace pour augmenter les teneurs en glucose sanguin chez un sujet vertébré lorsqu'il est ingéré par le sujet, le glucide étant présent en une quantité dans l'intervalle d'environ 50 % à environ 65 % de l'énergie fournie dans le repas ;
b) une source de graisse alimentaire, la graisse alimentaire étant présente en une quantité dans l'intervalle d'environ 15 % à environ 30 % de l'énergie fournie dans le repas ;
c) une source de protéine alimentaire, la protéine alimentaire étant présente en une quantité dans l'intervalle d'environ 10 % à environ 25 % de l'énergie fournie dans le repas ; et, éventuellement
d) une source de fibre alimentaire et d'agent aromatisant.

13. Utilisation selon la revendication 12, dans laquelle le kit est utilisé pour diagnostiquer un trouble du métabolisme des glucides.

14. Utilisation selon la revendication 13, dans laquelle le kit est utilisé pour diagnostiquer un trouble du métabolisme des glucides choisi dans le groupe consistant en diabète sucré, tolérance au glucose altérée, résistance à l'insuline, diabète non insulino-dépendant, diabète de l'adulte, diabète gestationnel et hyperinsulinémie.

15. Utilisation selon la revendication 12, dans laquelle le constituant du sang est le glucose.

16. Utilisation selon la revendication 12, dans laquelle le constituant du sang est l'insuline.

17. Utilisation selon la revendication 12, dans laquelle le kit est utilisé pour ajuster la posologie d'un médicament qui réduit la concentration de glucose post-prandiale.

18. Utilisation selon la revendication 16, dans laquelle le médicament est l'insuline.

19. Utilisation selon la revendication 12, dans laquelle le kit est utilisé pour l'autocontrôle et/ou l'autodiagnostic du diabète.
